(19) Europäisches Patentamt
European Patent Office
Office européen des brevets

(11) Publication number: 0 212 145 B1

# (12) EUROPEAN PATENT SPECIFICATION

(45) Date of publication of patent specification: 02.01.91 Bulletin 91/01

(51) Int. Cl.$^5$: A61K 31/72, C08B 37/00, C08B 11/145

(21) Application number: 86108708.8

(22) Date of filing: 26.06.86

## (54) Quaternary ammonium salts of natural polysaccharides possessing hypocholesterolemic activity.

The file contains technical information submitted after the application was filed and not included in this specification

(30) Priority: 14.08.85 IT 2193785

(43) Date of publication of application: 04.03.87 Bulletin 87/10

(45) Publication of the grant of the patent: 02.01.91 Bulletin 91/01

(84) Designated Contracting States: AT BE CH DE FR GB IT LI LU NL SE

(56) References cited:
EP-A- 0 066 135
EP-A- 0 115 574
GB-A- 1 136 842
US-A- 3 769 399
ABSTRACTS BULLETIN OF THE INSTITUTE OF PAPER CHEMISTRY, vol. 51, no. 5, November 1980, page 524, abstract no. 4779, Appleton, Wisconsin, US; & JP-A-79 87 786 (FUJI CHEMICAL CO. LTD.) 12-07-1979

(56) References cited:
CHEMICAL ABSTRACTS, vol. 91, no. 22, November 1979, page 90, abstract no. 176985t, Columbus, Ohio, US; & JP-A-79 105 190 (LION FAT AND OIL CO. LTD.) 17-08-1979
CHEMICAL ABSTRACTS, vol. 96, 1982, page 55, abstract no. 115788n, Columbus, Ohio, US; L.D. JOSHI et al.: "Dose variation effects of guar gum on blood sugar and serum cholesterol levels in experimental rats", & INDIAN J. PHARM. SCI. 1981, 43(5), 166-8
CHEMICAL ABSTRACTS, vol. 89, no. 7, 14th August 1978, page 485, abstract no. 58752a, Columbus, Ohio, US; K. TSUJI et al.: "Effects of polysaccharides on cholesterol metabolism. VII. Effects of various polysaccharide derivatives, lignin, and synthetic polymers on serum and liver cholesterol levels in rats", & EIYOGAKU ZASSHI 1977, 35(5), 227-34

(73) Proprietor: Etablissement TEXCONTOR
Egertstrasse 15
Vaduz (LI)

(72) Inventor: Conti, Franco
via della Spiga 33
Milano (IT)

(74) Representative: Gervasi, Gemma, Dr. et al
NOTARBARTOLO & GERVASI Srl 33, Viale Bianca Maria
I-21100 Milano (IT)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to quaternary ammonium salts of natural polysaccharides, possessing hypocholesterolemic activity.

More particularly, the invention relates to water-soluble quaternary ammonium salts of polysaccharides of vegetable origin, of advantageous use in human therapy as hypocholesterolemic agents, to the process for their production, and to the relative pharmaceutical compositions.

The polysaccharide ammonium salts according to the present invention have the following general formula :

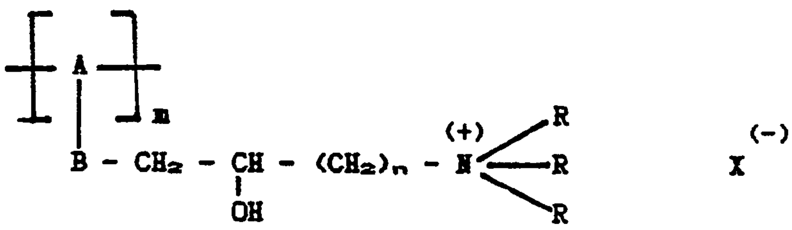

(I)

in which A represents the monomer unit of a natural polysaccharide, m is a whole number between 100 and 1000, n is a whole number between 0 and 10, B is O or S or NH or NR′ where R′ is an acyl and preferably an acetyl, R is a linear alkyl radical of 1-4 carbon atoms, and $X^{(-)}$ is $Cl^-$, $Br^-$, $I^-$, $HSO_4^-$, or $CH_3O\text{-}SO_3^-$, $NO_3^-$ or the negative ion of a pharmaceutically acceptable organic acid.

Generally, known products possessing hypocholesterolemic activity are cross-linked synthetic polymer derivatives, for example of polystyrene. These polymers are insoluble in water and therefore their availability is extremely low, and moreover they present undesirable side effects.

Of the natural polymers, only suitably treated chitosan is used as a product with hypocholesterolemic activity.

However, in the known art (M. Sugano, T. Fujikawa et al., Am. J. of Clinical Nutrition, 33, April 1980, pp. 787, 793), the positive charges on the polysaccharide macromolecules of the chitosan are obtained by protonising the amino group, so that under the pH conditions of the intestinal tract (pH 7.2) the proton is almost completely removed as the pKa of chitosan is 6.3. For this reason, the capacity of the polymer to interact with bile salts is very low.

In one of our previous patents (USA patent 4,436,731) the chitosan quaternisation reaction is conducted directly on the amino group present in the amino-glucoside ring, and the positive charge remains present under all pH and ionic force conditions of the gastro-intestinal tract, however its closeness to the aminoglucoside ring creates accessibility problems for the bile salt molecules.

A further known polymer containing quaternary ammonium groups and used as a hypocholesterolemic agent is obtained from dextran, as described for example in European patent application 66135.

Because of the extracelllular bacterial origin of dextran, this product is not very stable in the presence of the intestinal bacterial flora, and can therefore be easily broken down and assimilated.

The break-down of the dextran molecules, having a side-chain containing nitrogenated groups, can also give rise to products with a certain degree of toxicity, and is facilitated by the strongly acid environment of the stomach.

Both the assimilability and the potential toxicity of break-down products of dextran polymers containing quaternary ammonium groups make their use as hypocholesterolemic agents problematic.

We have now discovered new water-soluble quaternary ammonium salts of natural polysaccharides, in which the positive charge is at a suitable distance from the polysaccharide macromolecule, on a branch which is easily available for interaction with the bile salt molecules.

Said salts, besides exercising a very intense hypocholesterolemic activity, are substantially free from toxicity on oral administration and are also stable at the pH values corresponding to those existing in the human stomach.

The quaternary ammonium salts of polysaccharides possessing hypocholesterolemic activity according to the present invention are characterised by being of formula (I) with a degree of substitution of between 0.5 and 2. The term "degree of substitution" signifies the ratio of the number of moles of side chain containing the quaternary ammonium group to the number of monomer units of the polysaccharide. The preparation process is characterised by reacting a pretreated natural polysaccharide with a quaternary ammonium salt functionalised at one of its ends with an epoxy group able to react with nucleophilic groups of OH or SH or

$NH_2$ or NHR type present in the polysaccharide molecule and where R is a linear alkyl radical containing 1-10 carbon atoms, in a reaction medium consisting of a mixed solvent.

These and further characteristics, both relative to the process for preparing the quaternary salts according to the invention and relative to their therapeutic applications, will be more apparent from the description given hereinafter of preferred embodiments of the invention, by way of non-limiting example.

The polysaccharides preferably used are tragacanth, guar and carob gums, and cellulose, tamarind and chitosan. Said polysaccharides are among the most representative of those available, and can be either water-insoluble such as cellulose or chitosan, or water-soluble such as guar gum and carob gum.

Typically, according to the present invention, the natural polysaccharide to be reacted with the quaternary ammonium salt functionalised with the epoxy group is subjected to pretreatment with an acid, with a base or with a solvent in order to adjust its molecular weight to within the reqired range, and to increase its reactivity.

Water-insoluble polysaccharides are either pretreated by acid hydrolysis to obtain polymers with a suitable molecular weight of between 50,000 and 300,000, or are dissolved in suitable solvents (for example cellulose is dissolved in cupriethylenediamine and chitosan is dissolved in formic acid) and are then precipitated in an aqueous environment at pH 10-12 to obtain an almost amorphous polymer able to also react in the heterogeneous phase.

Water-soluble polysaccharides are pretreated with sodium hydroxide solutions of 10-30% concentration by weight, and are then dispersed in an organic solvent of for example the dioxane, acetone, ethyl alcohol or isopropyl alcohol type, to obtain NaOH-polysaccharide complexes which are very reactive towards the epoxy compounds.

The reaction between the polysaccharide and epoxy compound is conducted in a solvent under agitation at a temperature of between 40°C and 100°C for a time of between 4 and 16 hours, with a molar ratio of polysaccharide to epoxy compound of between 1 :1 and 1 :6 and a weight ratio of polysaccharide to reaction medium of between 1 :5 and 1 :35.

The degree of substitution is between 0.5 and 2.

The following examples and tables, relative respectively to the process for preparing compounds of general formula (I) and to their therapeutic applications, are given hereinafter for non-limiting illustrative purposes.

## EXAMPLE 1

100 g of commercial chitosan with a 20% degree of acetylation are dissolved in 5 litres of 0.1 M formic acid and the solution heated under reflux for 24 hours.

The solution is then cooled and precipitated by treating with 10 litres of 5 N NaOH, the precipitate is filtered off, washed with isopropyl alcohol until its $H_2O$ content is 30%, and is then dispersed in 600 ml of isopropyl alcohol.

The system is kept under agitation for 40 minutes and 262 g of glycidyltrimethyl ammonium chloride are then added, and the mixture is heated to 80°C for 6 hours.

The mixture is cooled and petroleum ether is added, the precipitate is filtered off, washed repeatedly with acetone and then dried under vacuum to obtain 255 g of dry product.

The degree of substitution, determined by $^1$H-NMR, is 1.5 and the water-solubility of the product is 12% by weight at 25°C.

## EXAMPLE 2

100 g of powdered guar gum are dispersed in 2 litres of 20 wt% NaOH solution, and the system kept under agitation at ambient temperature for 1 hour.

The system is then dispersed in 3 litres of dioxane, 95 g of glycidyltrimethyl ammonium chloride are added, and the mixture kept under agitation at 40°C for 24 hours.

The product is then precipitated by treatment with acetone, is washed repeatedly with acetone and petroleum ether, and is finally dried under vacuum to obtain 250 g of dry product.

The degree of substitution, determined by $^1$H-NMR, is 1.4 and the water-solubility is 15% by weight at 25°C.

## EXAMPLE 3

100 g of cellulose powder previously hydrolysed with 0.1 M HCl and swollen in 2000 ml of 0.5 M cupriethylenediamine at ambient temperature are regenerated in acetone.

The cellulose is repeatedly washed in mixtures of water and acetone, and is then immersed in an 8 wt% solution of NaOH and then squeeze-dried and dispersed in 2 litres of 95% ethanol. 150 g of propyleneoxidetriethylammonium bromide are added and the mixture heated to 80°C for 24 hours.

After cooling, the product is precipitated by treatment with petroleum ether, is filtered off, washed with acetone and then dried under vacuum.

The degree of substitution, determined by $^1$H-NMR, is 0.9 and the water-solubility 7% by weight at 25°C.

Using the aforesaid mehtods, quaternary ammonium salts were prepared from powdered carob gum, tamarind and tragacanth.

The products according to the invention were used in a series of pharmacological trials, the results of which are summarised in the following tables. For comparison purposes, the results are also given of trials carried out under the same conditions with cholestyramine, which is the most effective resin of hypocholesterolemic activity currently available.

The following tests were used to demonstrate the "in vivo" hypocholesterolemic effect of the various resins :

1) Action on hypercholesterolemia induced in the rat and rabbit by a cholesterol-enriched diet

2) Action on the fecal excretion of bile acids in the dog.

1) To induce hypercholesterolemia in rats, the animals were kept under a Nath diet (Nath and coll., J. Nutrit. 67, 289, 1959) containing :

| | | |
|---|---|---|
| - | devitaminised casein | 20% |
| - | DL-methionine | 0.4% |
| - | Hegsted saline mixture | 4% |
| - | Saccharose | 49.1% |
| - | Cholesterol | 1% |
| - | Cholic acid | 0.5% and vitamins |

Hypercholesterolemia was induced in the rabbits by administering 1 g/animal/day of cholesterol with a gastric probe.

Sprague-Dawley rats with an average weight of 200 g and New Zealand rabbits weighing 3 kg were used, divided into groups of 10 animals each.

All the animals were made hypercholesterolemic by means of the diet. One group remained untreated and served as the controls, whereas the other groups were treated with 0.5 g/kg of the various products under examination for 30 days.

After 30 days of treatment all the animals were sacrificed and the total plasmatic cholesterol in the blood collected from the carotid arteries was determined (Pearson and coll., J. Chim. Endocrin. Metabolism 12, 1245, 1952).

2) In order to evaluate the fecal excretion of bile acids, 48 male beagle dogs of approximately 8 kg weight were used, divided into 12 groups of 4 animals each. All the animals were kept under diet and stalled under standard conditions, and with the exception of one group of control dogs all the other groups were given in addition to the diet 2 g/kg/day of one of the products under examination for 25 days.

On the 26th day after the commencement of the trial, the feces of the dogs, which had been kept under fast for 12 hours in a metabolic cage, were subjected to bile acid determination (Grundy and coll., J. Lipid Res. 6, 397, 1965 ; Makita and coll., Ann. Biochem. 5, 523, 1963 ; Forman and coll., Clin. Chem. 14, 348, 1969).

Tables 1 and 2 summarise the results obtained on the rats and rabbits made hypercholesterolemic by diet, and treated with the various indicated products. Table 3 shows the values of the bile acid excretions of dogs treated with 2 g/kg/day of the various compounds.

The products according to the invention used in these trials are as follows :

ET 1015 = product with guar gum substrate
ET 1016 = product with powdered carob gum substrate
ET 1018 = product with cellulose substrate
ET 1019 = product with tamarind substrate
ET 1020 = product with chitosan substrate

ET 1021 = product with tragacanth substrate.

TABLE 1

Total serous cholesterol values in rats subjected to a Nath diet (Nath and coll., J. Nutrit. 67, 289, 1959) for 30 days and treated with the various products.

| | Control | Chole-styramine | ET1015 | ET1016 | ET1018 | ET1019 | ET1020 | ET1021 |
|---|---|---|---|---|---|---|---|---|
| No. rats | 10 | 10 | 10 | 10 | 10 | 10 | 10 | 10 |
| mg % | 312 | 148 | 121 | 124 | 103 | 134 | 90 | 129 |
| | ±24.7 | ±11.9 | ±10.7 | ±9.4 | ±6.8 | ±9.7 | ±9.1 | ±10.3 |

TABLE 2

Total serous cholesterol values in rabbits subjected to a cholesterol-enriched diet for 30 days and treated with the various products.

| | Control | Chole-styramine | ET1015 | ET1016 | ET1018 | ET1019 | ET1020 | ET1021 |
|---|---|---|---|---|---|---|---|---|
| No. rabbits | 10 | 10 | 10 | 10 | 10 | 10 | 10 | 10 |
| mg % | 702 | 269 | 210 | 122 | 184 | 229 | 172 | 208 |
| | ±57.6 | ±18.2 | ±16.5 | ±15.9 | ±13.9 | ±15.1 | ±14.8 | ±15.1 |

TABLE 3

Fecal bile acid excretion in dogs treated with the various products for 25 days.

| | Control | Chole-styramine | ET1015 | ET1016 | ET1018 | ET1019 | ET1020 | ET1021 |
|---|---|---|---|---|---|---|---|---|
| No. dogs | 4 | 4 | 4 | 4 | 4 | 4 | 4 | 4 |
| mcg/g | 785 | 2098 | 2540 | 2580 | 2890 | 2350 | 2950 | 2755 |
| of feces | ±47.4 | ±132.6 | ±146.3 | ±150.8 | ±162.7 | ±145.3 | ±178.5 | ±166.4 |

From the aforegoing tables it is clear that the quaternary ammonium salts of polysaccharides according to the present invention have greater hypocholesterolemic activity than cholestyramine both in rats and in rabbits subjected to a hypercholesterolemic diet.

The results obtained for fecal excretion in the dog confirm the binding activity of the products according to the invention towards bile acids. In this respect, administering these products leads to large increases in the amount of bile acids excreted with the feces, to a considerably greater extent than with equivalent doses of cholestyramine.

**Claims**

**Claims for the Contracting States : BE CH DE FR GB IT LI LU NL SE**

1. Water-soluble quaternary ammonium salts of natural polysaccharides possissing hypocholesterolemic activity, of the following general formula :

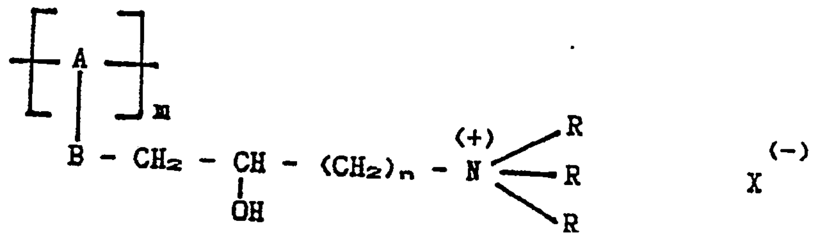

(I)

in which A represents the monomer unit of a natural polysaccharide, m is a whole number between 100 and 1000, n is a whole number between 0 and 10, B is O or S or NH or NR′ where R′ is an acyl and preferably an acteyl, R is a linear alkyl radical of 1-4 carbon atoms, and $X^{(-)}$ is $Cl^-$ $Br^-$, $I^-$, $HSO_4^-$, $CH_3O\text{-}SO_3^-$, $NO_3^-$ or the negative ion of a pharmaceutically acceptable organic acide, and with a degree of subsitution of between 0.5 and 2 and a molecular weight of betweein 50,000 and 300,000.

2. Water-soluble quaternary ammonium salts of natural polysaccharides as claimed in claim 1, wherein the natural polysaccharide is tragacanth.

3. Water-soluble quaternary ammonium salts of natural polysaccharides as claimed in claim 1, wherein the natural polysaccharide is guar gum.

4. Water-soluble quaternary ammonium salts of natural polysaccharides as claimed in claim 1, wherein the natural polysaccharide is carob gum.

5. Water-soluble quaternary ammonium salts of natural polysaccharides as claimed in claim 1, wherein the natural polysaccharide is cellulose.

6. Water-soluble quaternary ammonium salts of natural polysaccharides as claimed in claim 1, wherein the natural polysaccharide is tamarind.

7. Water-soluble quaternary ammonium salts of natural polysaccharides as claimed in claim 1, wherein the natural polysaccharide is chitosan.

8. A process for preparing quaternary ammonium salts of polysaccharides possessing hypocholesterolemic activity, of the following general formula :

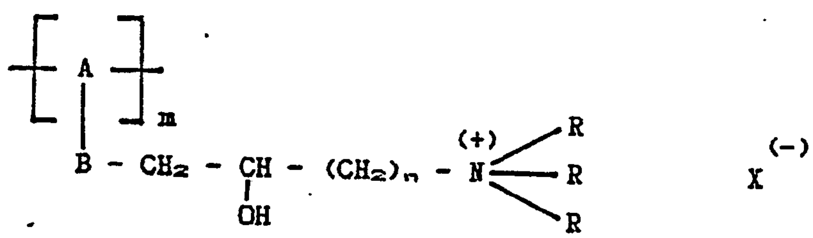

(I)

in which A represents the monomer unit of a natural polysaccharide, m is a whole number between 100 and 1000, n is a whole number between 0 and 10, B is O or S or NH or NR′ where R′ is an acyl and preferably an acetyl, R is a linear alkyl radical of 1-4 carbon atoms, and $X^{(-)}$ is $Cl^-$, $Br^-$, $I^-$, $HSO_4^-$, $CH_3O\text{-}SO_3^-$, $NO_3^-$ or the negative ion of a pharmaceutically acceptable organic acid, and with a degree of substitution of between 0.5 and 2, characterised by reacting a pretreated natural polysaccharide with a quaternary ammonium salt functionalised at one of its ends with an epoxy group either pre-existing or formed in situ and able to react with nucleophilic groups of OH or SH or $NH_2$ or NHR type present in the polysaccharide molecule and where R has the aforesaid meaning, in a reaction medium consisting of a mixed solvent.

9. A process as claimed in claim 8, characterised in that said natural polysaccharides are tragacanth, guar gum, carob gum, cellulose, tamarind and chitosan.

10. A process as claimed in claim 8, characterised in that said natural polysaccharides, if insoluble in water, are previously hydrolysed in an acid environment to obtain polymers with a molecular weight of between 50,000 and 300 000.

11. A process as claimed in claim 8, characterised in that said natural polysaccharides, if insoluble in water, are previously activated by dissolving in a suitable solvent and then precipitating in an aqueous environment of pH between 10 and 12.

12. A process as claimed in claim 8, characterised in that said natural polysaccharides, if soluble in water, are previously activated by treatment with sodium hydroxide solutions of between 10 and 30% concentration by weight, and are then dispersed in an organic solvent such as dioxane, acetone, ethyl alcohol or isopropyl alcohol.

13. A process as claimed in claim 8, characterised in that said reaction is conducted under agitation at a temperature of between 40°C and 100°C for a time of between 4 and 16 hours.

14. A process as claimed in claim 8, characterised in that said reaction is conducted using a molar ratio of polysaccharide to epoxy compound of between 1 :1 and 1 :6 and a weight ratio of polysaccharide to reaction medium of between 1 :5 and 1 :35.

15. Therapeutic compositions of hypocholesterolemic activity characterised by comprising a product of formula (I) as active principle.

**Claims for the Contracting State AT**

1. A process for preparing quaternary ammonium salts of polysaccharides possessing hypocholesterolemic activity, of the following general formula :

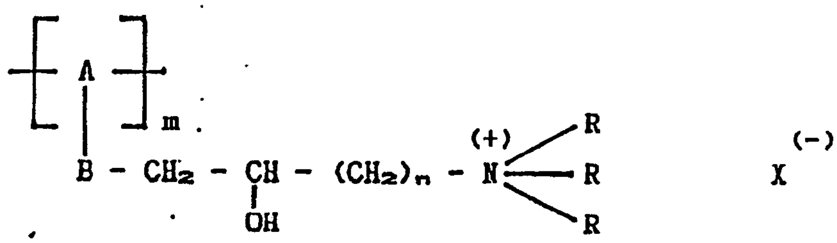

(I)

in which A represents the monomer unit of a natural polysaccharide, m is a whole number between 100 and 1000, n is a whole number between 0 and 10, B is O or S or NH or NR′ where R′ is an acyl and preferably an acetyl, R is a linear alkyl radical of 1-4 carbon atoms, and $X^{(-)}$ is $Cl^-$, $Br^-$, $I^-$, $HSO_4^-$, $CH_3O\text{-}SO_3^-$, $NO_3^-$ or the negative ion of a pharmaceutically acceptable organic acid, and with a degree of substitution of between 0.5 and 2, characterised by reacting a pretreated natural polysaccharide with a quaternary ammonium salt funactionalised at one of its ends with an epoxy group either pre-existing or formed in situ and able to react with nucleophilic groups of OH or SH or $NH_2$ or NHR type present in the polysaccharide molecule and where R has the aforesaid meaning, in a reaction medium consisting of a mixed solvent.

2. A process as claimed in claim 1, characterised in that said natural polysaccharides are tragacanth, guar gum, carob gum, cellulose, tamarind and chitosan.

3. A process as claimed in claim 1, characterised in that said natural polysaccharides, if insoluble in water, are previously hydrolysed in an acid ennvironment to obtain polymers with a molecular weight of between 50,000 and 300,000.

4. A process as claimed in claim 1, characterised in that said natural polysaccharides, if insoluble in water, are previously activated by dissolving in a suitable solvent and then precipitating in an aqueous environment of pH between 10 and 12.

5. A process as claimed in claim 1, characterised in that said natural polysaccharides, if soluble in water, are previously activated by treatment with sodium hydroxide solutions of between 10 and 30% concentration by weight, and are then dispersed in an organic solvent such as dioxane, acetone, ethyl alcohol or isopropyl alcohol.

6. A process as claimed in claim 1, characterised in that said reaction is conducted under agitation at a temperature of between 40°C and 100°C for a time of between 4 and 16 hours.

7 . A process as claimed in claim 1, characterised in that said reaction is conducted using a molar ratio of polysaccaride to epoxy compound of between 1 :1 and 1 :6 and a weight ratio of polysaccharide to reaction medium of between 1 :5 aid 1 :35.

## Ansprüche

**Patentansprüche für die Vertragsstaaten : BE, CH, DE, FR, GB, IT, LI, LU, NL, SE**

1. Wasserlösliche quaternäre Ammoniumsalze von natürlichen Polysacchariden mit hypocholesterinämischer Aktivität der folgenden allgemeinen Formel :

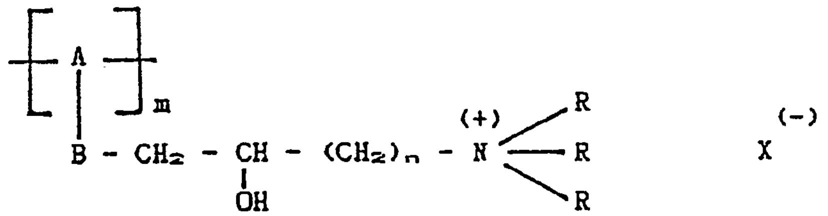 (I),

worin A die Monomereinheit eines natürlichen Polysaccharids darstellt, m eine ganze Zahl von 100 bis 1000 ist, n eine ganze Zahl von Null bis 10 ist, B die Bedeutung O, S, NH oder NR′ hat, wobei R′ Acyl, vorzugsweise Acetyl ist, R einen linearen Alkylrest mit 1 bis 4 Kohlenstoffatomen bedeutet und $X^{(-)}$ $Cl^-$, $Br^-$, $J^-$, $HSO_4^-$, $CH_3O\text{-}SO_3^-$, $NO_3^-$ oder das negative Ion einer pharmazeutisch annehmbaren organischen Säure ist, und mit einem Substitutionsgrad von 0,5 bis 2 und einem Molgewicht von 50 000 bis 300 000.

2. Wasserlösliche quaternäre Ammoniumsalze von natürlichen Polysacchariden nach Anspruch 1, worin das natürliche Polysaccharid Traganth ist.

3. Wasserlösliche quaternäre Ammoniumsalze von natürlichen Polysacchariden nach Anspruch 1, worin das natürliche Polysaccharid Guargummi ist.

4. Wasserlösliche quaternäre Ammoniumsalze von natürlichen Polysacchariden nach Anspruch 1, worin das natürliche Polysaccharid Johannisbrotgummi ist.

5. Wasserlösliche quaternäre Ammoniumsalze von natürlichen Polysacchariden nach Anspruch 1, worin das natürliche Polysaccharid Cellulose ist.

6. Wasserlösliche quaternäre Ammoniumsalze von natürlichen Polysacchariden nach Anspruch 1, worin das natürliche Polysaccharid Tamarinde ist.

7. Wasserlösliche quaternäre Ammoniumsalze von natürlichen Polysacchariden nach Anspruch 1, worin das natürliche Polysaccharid Chitosan ist.

8. Verfahren zur Herstellung von wasserlöslichen quaternären Ammoniumsalzen von Polysacchariden mit hypocholesterinämischer Aktivität der folgenden allgemeinen Formel :

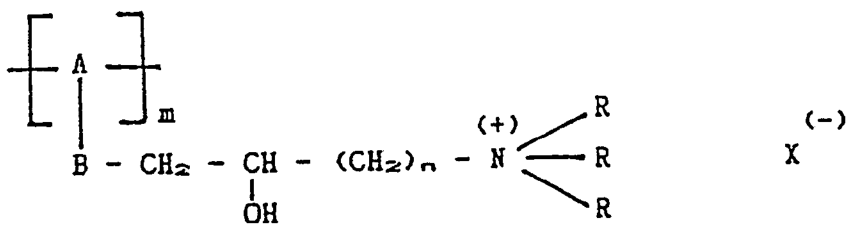 (I),

worin A die Monomereinheit eines natürlichen Polysaccharids darstellt, m eine ganze Zahl von 100 bis 1000 ist, n eine ganze Zahl von Null bis 10 ist, B die Bedeutung O, S, NH oder NR′ hat, wobei R′ Acyl, vorzugsweise Acetyl ist, R einen linearen Alkylrest mit 1 bis 4 Kohlenstoffatomen bedeutet und $X^{(-)}$ $Cl^-$, $Br^-$, $J^-$, $HSO_4^-$, $CH_3O\text{-}SO_3^-$, $NO_3^-$ oder das negative Ion einer pharmazeutisch annehmbaren organischen Säure ist, und mit einem Substitutionsgrad von 0,5 bis 2, dadurch gekennzeichnet, daß ein vorbehandeltes natürliches Polysaccharid mit einem an einem seiner Enden mit einer entweder bereits vorhandenen oder in situ gebildeten, zur Umsetzung mit im Polysaccharidmolekül vorhandenen nucleophilen Gruppen vom OH-, SH-, $NH_2$- oder NHR-Typ fähigen Epoxygruppe funktionalisierten quaternären Ammoniumsalz, wobei R die obgenannte Bedeutung hat, in einem Reaktionsmedium, bestehend aus einem gemischten Lösungsmittel, umgesetzt wird.

9. Verfahren nach Anspruch 8, dadurch gekennzeichnet, daß die natürlichen Polysaccharide Traganth, Guargummi, Johannisbrotgummi, Cellulose, Tamarinde oder Chitosan sind.

10. Verfahren nach Anspruch 8, dadurch gekennzeichnet, daß die natürlichen Polysaccharide, wenn sie in Wasser unlöslich sind, vorher in einer sauren Umgebung hydrolysiert werden, um Polymere mit einem Molgewicht von 50 000 bis 300 000 zu erhalten.

11. Verfahren nach Anspruch 8, dadurch gekennzeichnet, daß die natürlichen Polysaccharide, wenn sie in Wasser unlöslich sind, vorher durch Lösen in einem geeignetem Lösungsmittel und anschließendes Ausfällen in einer wässerigen Umgebung mit einem pH von 10 bis 12 aktiviert werden.

12. Verfahren nach Anspruch 8, dadurch gekennzeichnet, daß die natürlichen Polysaccharide, wenn sie in Wasser löslich sind, vorher durch Behandlung mit 10 bis 30 gew.%igen Natriumhydroxydlösungen aktiviert und dann in einem organischen Lösungsmittel, wie Dioxan, Aceton, Ethylalkohol oder Isopropylalkohol, dispergiert werden.

13. Verfahren nach Anspruch 8, dadurch gekennzeichnet, daß die Umsetzung unter Bewegung bei einer Temperatur von 40 bis 100°C während einer Zeitdauer von 4 bis 16 Stunden durchgeführt wird.

14. Verfahren nach Anspruch 8, dadurch gekennzeichnet, daß die Umsetzung unter Verwendung eines Molverhältnisses von Polysaccharid zu Epoxyverbindung von 1 :1 bis 1 :6 und eines Gewichtsverhältnisses von Polysaccharid zu Reaktionsmedium von 1 :5 bis 1 :35 durchgeführt wird.

15. Therapeutische Zusammensetzungen mit hypocholesterinämischer Aktivität, dadurch gekennzeichnet, daß sie ein Produkt der Formel (I) als Wirkstoff umfassen.

**Patentansprüche für der Vertragsstaat : AT**

1. Verfahren zur Herstellung von wasserlöslichen quaternären Ammoniumsalzen von Polysacchariden mit hypocholesterinämischer Aktivität der folgenden allgemeinen Formel :

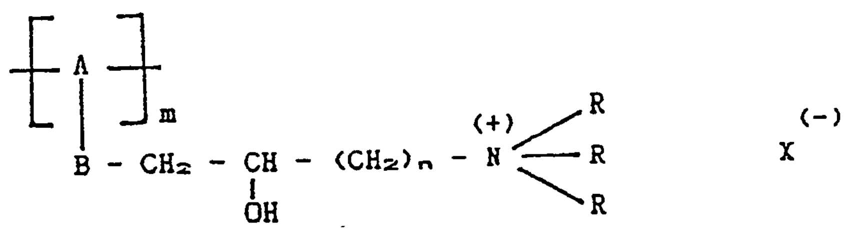 (I),

worin A die Monomereinheit eines natürlichen Polysaccharids darstellt, m eine ganze Zahl von 100 bis 1000 ist, n eine ganze Zahl von Null bis 10 ist, B die Bedeutung O, S, NH oder NR' hat, wobei R' Acyl, vorzugsweise Acetyl ist, R einen linearen Alkylrest mit 1 bis 4 Kohlenstoffatomen bedeutet und $X^{(-)}$ $Cl^-$, $Br^-$, $J^-$, $HSO_4^-$, $CH_3O\text{-}SO_3^-$, $NO_3^-$ oder das negative Ion einer pharmazeutisch annehmbaren organischen Säure ist, und mit einem Substitutionsgrad von 0,5 bis 2, dadurch gekennzeichnet, daß ein vorbehandeltes natürliches Polysaccharid mit einem an einem seiner Enden mit einer entweder bereits vorhandenen oder in situ gebildeten, zur Umsetzung mit im Polysaccharidmolekül vorhandenen nucleophilen Gruppen vom OH-, SH-, $NH_2$- oder NHR-Typ fähigen Epoxygruppe funktionalisierten quaternären Ammoniumsalz, wobei R die obgenannte Bedeutung hat, in einem Reaktionsmedium, bestehend aus einem gemischten Lösungsmittel, umgesetzt wird.

2. Verfahren nach Anspruch 1, dadurch gekennzeichnet, daß die natürlichen Polysaccharide Traganth, Guargummi, Johannisbrotgummi, Cellulose, Tamarinde oder Chitosan sind.

3. Verfahren nach Anspruch 1, dadurch gekennzeichnet, daß die natürlichen Polysaccharide, wenn sie in Wasser unlöslich sind, vorher in einer sauren Umgebung hydrolysiert werden, um Polymere mit einem Molgewicht von 50 000 bis 300 000 zu erhalten.

4. Verfahren nach Anspruch 1, dadurch gekennzeichnet, daß die natürlichen Polysaccharide, wenn sie in Wasser unlöslich sind, vorher durch Lösen in einem geeignetem Lösungsmittel und anschließendes Ausfällen in einer wässerigen Umgebung mit einem pH von 10 bis 12 aktiviert werden.

5. Verfahren nach Anspruch 1, dadurch gekennzeichnet, daß die natürlichen Polysaccharide, wenn sie in Wasser löslich sind, vorher durch Behandlung mit 10 bis 30 gew.%igen Natriumhydroxydlösungen aktiviert und dann in einem organischen Lösungsmittel wie Dioxan, Aceton, Ethylalkohol oder Isopropylalkohol, dispergiert werden.

6. Verfahren nach Anspruch 1, dadurch gekennzeichnet, daß die Umsetzung unter Bewegung bei einer Temperatur von 40 bis 100°C während einer Zeitdauer von 4 bis 16 Stunden durchgeführt wird.

7. Verfahren nach Anspruch 1, dadurch gekennzeichnet, daß die Umsetzung unter Verwendung eines Molverhältnisses von Polysaccharid zu Epoxyverbindung von 1 :1 bis 1 :6 und eines Gewichtsverhältnisses von Polysaccharid zu Reaktionsmedium von 1 :5 bis 1 :35 durchgeführt wird.

**Revendications**

**Revendications pour les Etats Contractants : BE CH DE FR GB IT LI LU NL SE**

1. Sels d'ammonium quaternaire de polysaccharides naturels hydrosolubles, possédant une activité hypocholestérolémique, de formule générale suivante :

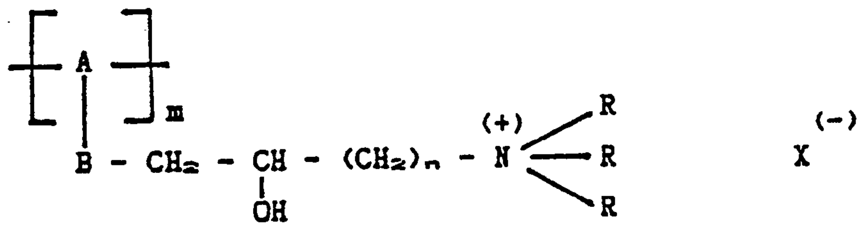 (I)

dans laquelle A représente le motif monomère d'un polysaccharide naturel,
m est un nombre entier compris entre 100 et 1000,
n est un nombre entier compris entre 0 et 10,
B est l'oxygène ou le soufre ou NH ou NR' avec R' qui représente un radical acyle et de préférence acétyle,
R est un radical alkyle linéaire avec 1 à 4 atomes de carbone, et $X^{(-)}$ est $Cl^-$, $Br^-$, $I^-$, $HSO_4^-$, $CH_3O\text{-}SO_3^-$, $NO_3^-$ ou l'ion négatif d'un acide organique acceptable pharmaceutiquement, et avec un degré de substitution compris entre 0,5 et 2 et un poids moléculaire compris entre 50.000 et 300.000.

2. Sels d'ammonium quaternaire de polysaccharides naturels hydrosolubles, tels que revendiqués dans la revendication 1, dans lesquels le polysaccharide naturel est la gomme adragante.

3. Sels d'ammonium quaternaire de polysaccharides naturels hydrosolubles, tels que revendiqués dans la revendication 1, dans lesquels le polysaccharide est la gomme de guar.

4. Sels d'ammonium quaternaire de polysaccharides naturels hydrosolubles, tels que revendiqués dans la revendication 1, dans lesquels le polysaccharide est la gomme de caroube.

5. Sels d'ammonium quaternaire de polysaccharides naturels hydrosolubles, tels que revendiqués dans la revendication 1, dans lesquels le polysaccharide est la cellulose.

6. Sels d'ammonium quaternaire de polysaccharides naturels hydrosolubles, tels que revendiqués dans la revendication 1, dans lesquels le polysaccharide est le tamarin.

7. Sels d'ammonium quaternaire de polysaccharides naturels hydrosolubles, tels que revendiqués dans la revendication 1, dans lesquels le polysaccharide est le chitosane.

8. Procédé de préparation des sels d'ammonium quaternaire de polysaccharides possédant une activité hypocholestérolémique, de formule générale suivante :

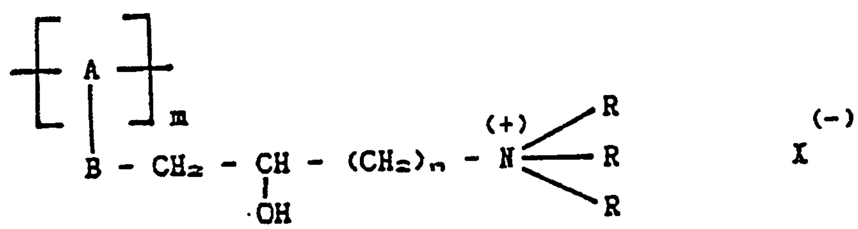 (I)

dans laquelle A représente le motif monomère d'un polysaccharide naturel,
m est un nombre entier compris entre 100 et 1000,
n est un nombre entier compris entre 0 et 10,
B est l'oxygène ou le soufre ou NH ou NR' avec R' qui représente un radical acyle et de préférence acétyle,
R est un radical alkyle linéaire avec 1 à 4 atomes de carbone, et $X^{(-)}$ est $Cl^-$, $Br^-$, $I^-$, $HSO_4^-$, $CH_3O\text{-}SO_3^-$, $NO_3^-$, ou l'ion négatif d'un acide organique acceptable pharmaceutiquement, et avec un degré de substitution compris entre 0,5 et 2, caractérisé par le fait que l'on fait réagir un polysaccharide naturel prétraité sur un sel d'ammonium quaternaire fonctionnalisé à l'une de ses extrémités avec un groupe époxy soit préexistant soit formé in situ et susceptible de réagir sur des groupes nucléophiles de type OH ou SH ou $NH_2$ ou NHR, présents dans la molécule de polysaccharide et où R a la signification indiquée ci-dessus, dans un milieu réactionnel constitué par un mélange de solvants.

9. Procédé tel que revendiqué dans la revendication 8, caractérisé par le fait que les polysaccharides naturels sont : la gome adragante, la gomme guar, la gomme de caroube, la cellulose, le tamarin et le chitosane.

10. Procédé tel que revendiqué dans la revendication 8, caractérisé par le fait que les polysaccharides naturels, s'ils sont insolubles dans l'eau, sont hydrolysés au préalable dans un milieu acide pour obtenir des polymères ayant un poids moléculaire compris entre 50.000 et 300.000.

11. Procédé tel que revendiqué dans la revendication 8, caractérisé par le fait que les polysaccharides

naturels, s'ils sont insolubles dans l'eau, sont activés au préalable par dissolution dans un solvant approprié et ensuite précipités dans un milieu aqueux dont le pH est compris entre 10 et 12.

12. Procédé tel que revendiqué dans la revendication 8, caractérisé par le fait que les polysaccharides naturels, s'ils sont solubles dans l'eau, sont activés au préalable par traitement dans des solutions d'hydroxyde de sodium de concentration comprise entre 10% et 30% en poids, et sont ensuite dispersés dans un solvant organique tel que le dioxane, l'acétone, l'alcool éthylique ou l'alcool isopropylique.

13. Procédé tel que revendiqué dans la revendication 8, caractérisé par le fait que la réaction est menée sous agitation, à une température comprise entre 40°C et 100°C pendant un temps compris entre 4 heures et 16 heures.

14. Procédé tel que revendiqué dans la revendication 8, caractérisé par le fait que la réaction est menée en utilisant un rapport molaire entre le polysaccharide et le composé époxy compris entre 1/1 et 1/6 et un rapport pondéral du polysaccharide au milieu réactionnel compris entre 1/5 et 1/35.

15. Compositions thérapeutiques ayant une activité hypocholestérolémique, caractérisées par le fait qu'elles contiennent un produit de formule (I) en tant que principe actif.

**Revendications pour l'Etat Contractant : AT**

1. Procédé de préparation de sels d'ammonium quaternaire de polysaccharides possédant une activité hypocholestérolémique, de formule générale suivante :

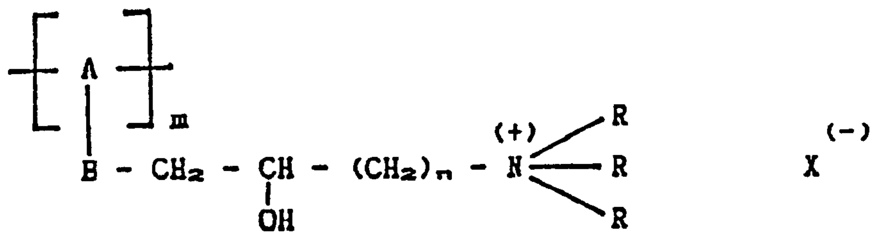

(I)

dans laquelle A représente le motif monomère d'un polysaccharide naturel,
m est un nombre entier compris entre 100 et 1000,
n est un nombre entier compris entre 0 et 10,
B est l'oxygène ou le soufre ou NH ou NR' avec R' qui représente un radical acyle et de préférence acétyle,
R est un radical alkyle linéaire avec 1 à 4 atomes de carbone, et $X^{(-)}$, est $Cl^-$, $Br^-$, $I^-$, $HSO_4^-$, $CH_3O\text{-}SO_3^-$, $NO_3^-$, ou l'ion négatif d'un acide organique acceptable pharmaceutiquement, et avec un degré de substitution compris entre 0,5 et 2, caractérisé par le fait que l'on fait réagir un polysaccharide naturel prétraité sur un sel d'ammonium quaternaire fonctionnalisé à l'une de ses extrémités avec un groupe époxy soit préexistant soit formé in situ et susceptible de réagir sur des groupes nucléophiles de type OH ou SH ou $NH_2$ ou NHR, présents dans la molécule de polysaccharide et où R a la signification indiquée ci-dessus, dans un milieu réactionnel constitué par un mélange de solvants.

2. Procédé tel que revendiqué dans la revendication 1, caractérisé par le fait que les polysaccharides naturels sont : la gome adragante, la gomme guar, la gomme de caroube, la cellulose, le tamarin et le chitosane.

3. Procédé tel que revendiqué dans la revendication 1, caractérisé par le fait que les polysaccharides naturels, s'ils sont insolubles dans l'eau, sont hydrolysés au préalable dans un milieu acide pour obtenir des polymères ayant un poids moléculaire compris entre 50.000 et 300.000.

4. Procédé tel que revendiqué dans la revendication 1, caractérisé par le fait que les polysaccharides naturels, s'ils sont insolubles dans l'eau, sont activés au préalable par dissolution dans un solvant approprié et ensuite précipités dans un milieu aqueux dont le pH est compris entre 10 et 12.

5. Procédé tel que revendiqué dans la revendication 1, caractérisé par le fait que les polysaccharides naturels, s'ils sont solubles dans l'eau, sont activés au préalable par traitement dans des solutions d'hydroxyde de sodium de concentration comrpise entre 10% et 30% en poids, et sont ensuite dispersés dans un solvant organique tel que le dioxane, l'acétone, l'alcool éthylique ou l'alcool isopropylique.

6. Procédé tel que revendiqué dans la revendication 1, caractérisé par le fait que la réaction est menée sous agitation, à une température comprise entre 40°C et 100°C pendant un temps compris entre 4 heures et 16 heures.

7. Procédé tel que revendiqué dans la revendication 1, caractérisé par le fait que la réaction est menée en utilisant un rapport molaire entre le polysaccharide et le composé époxy compris entre 1/1 et 1/6 et un

rapport pondéral du polysaccharide au milieu réactionnel compris entre 1/5 et 1/35.